Europäisches Patentamt

European Patent Office

Office européen des brevets

(11) Veröffentlichungsnummer: **0 210 667**

**A1**

## EUROPÄISCHE PATENTANMELDUNG

(21) Anmeldenummer: 86111484.1

(22) Anmeldetag: 07.12.84

(51) Int. Cl.4 **B65D 51/00**

(30) Priorität: 22.12.83 DE 3346351

(43) Veröffentlichungstag der Anmeldung:
**04.02.87 Patentblatt 87/06**

(60) Publication number of the original application in accordance with Art.76 EPC: 0 148 426

(84) Benannte Vertragsstaaten:
**AT BE CH DE FR GB IT LI LU NL SE**

(71) Anmelder: **Pharma-Gummi Wimmer West GmbH**
**Stolberger Strasse 21-41**
**D-5180 Eschweiler(DE)**

(72) Erfinder: **Wimmer, Hans**
**An Adamshäuschen 13**
**D-5100 Aachen(DE)**

(74) Vertreter: **Schmitt, Hans, Dipl.-Ing. et al**
**Patentanwälte Dipl.-Ing H. Schmitt Dipl.-Ing.**
**W. Maucher Dreikönigstrasse 13**
**D-7800 Freiburg(DE)**

(54) **Pharmazeutischer Stopfen und Verfahren zum Herstellen von pharmazeutischen Stopfen.**

(57) Ein pharmazeutischer Stopfen (2) zum Verschließen einer Flasche, eines medizinischen, pharmazeutischen od. dgl. Behälters, insbesondere einer Medikamentenflasche (1) weist in einem dem Behälterinnenraum (6) zugewandten, mindestens teilweise an der Behälterinnenwand (7) anliegenden Bereich einen fluorierten Polymer-od. dgl. Inertfilm auf. Dieser Film (8), der den Stopfen gegenüber dem Behälterinhalt weitesgehend chemisch neutral macht, haftet fest auf dem jeweiligen dem Behälterinnenraum zugewandten Stopfeninnenteil - (40), wobei diese Haftverbindung im Durchstichbereich für einen Infusionsdorn (60) od. dgl. unterbrochen ist. Um diesen Stopfen (2) herzustellen, wird die Haftfähigkeit des Filmes (8) im Durchstichbereich für einen Infusionsdorn (60) od. dgl. vor dem Aufbringen des Filmes (8) auf das gummielastische Stopfenteil (40) beseitigt. Hierdurch wird auch nach dem Durchstechen des Stopfens mit einem Infusionsdorn (60) ein ausreichend sicherer Wiederverschluß der Stopfens (2) erreicht.

Fig. 1

## Pharmazeutischer Stopfen und Verfahren zum Herstellen von pharmazeutischen Stopfen

Die Erfindung betrifft einen pharmazeutischen Stopfen zum Verschließen einer Flasche. eines medizinischen oder pharmazeutischen od. dgl. Behälters, insbesondere einer Medikamentenflasche. wobei der Stopfen od. dgl. in einem dem Behälterinnenraum zugewandten, mindestens teilweise an der Behälterinnenwand anliegenden Bereich einen fluorierten Polymer-od. dgl. Inertfilm aufweist und der Stopfenhals mit einem weiteren Bereich unmittelbar an der Wand der Behältermündung anliegt. .

Zum Abdichten und Verschließen von Medikamentenflaschen werden Stopfen von unterschiedlicher Form und unterschiedlichem Werkstoff eingesetzt (z. B. DE-PS 19 01 239, DE-PS 19 46 566). Um die chemische Neutralität der nach innen weisenden Stopfenfläche zu gewährleisten, sind deshalb chemisch sehr inerte und widerstandsfähige fluorierte Polymerfilme entwickelt worden, die auf die zu schützende Fläche des i. d. R. aus natürlichem oder synthetischem Gummi, gummielastischen oder aus reinen Thermoplasten bestehenden Stopfenkörpers aufgebracht werden können (z. B. DE-OS 21 46 421).

Probleme ergeben sich bei diesem Stopfenaufbau dann, wenn der Stopfen z. B. zur Entnahme des Behälterinhaltes mit einer Injektionsnadel einmal oder mehrfach durchstochen wird. Denn in diesem Fall kann der Polymerfilm od. dgl. Inertfilm verhindern, daß sich der durch die Injektionsnadel entstandene, dem Behälterinnern zugewandte Schlitz im gummielastischen Körper des Stopfens nicht mehr richtig schließt, der Stopfen somit undicht wird und der im Behälter noch vorhandene Inhalt praktisch nicht mehr gelagert werden kann. Wenn zudem nach dem ersten Durchstechen des Stopfens mit einem Infusionsdorn und nach dessen Entfernen mit dem Präparat z. B. Mischbewegungen durchgeführt werden oder das Präparat Gasdruck infolge chemischer Reaktionen entwickelt, kann gegebenenfalls Flüssigkeit bzw. Medikament-Anteil beim Schütteln oder durch Überdruck durch die Durchstichstelle nach außen treten. Dies kann u. U. in mehrfacher Hinsicht nachteilig sein. Z.B. ist die Dosierung des Medikamentes nicht mehr sicher. Auch kann der Flascheninhalt chemisch unerwünschte Auswirkungen auf die handhabende Person haben.

Es besteht daher die Aufgabe, einen pharmazeutischen Stopfen zu schaffen, der die entstandenen Durchstichlöcher selbsttätig wieder verschließt, ohne daß auf den Neutralitätsschutz durch den Polymerfilm od. dgl. Inertfilm verzichtet werden müßte.

Die Aufgabe wird dadurch gelöst. daß die Flächenverbindung zwischen dem fluorierten Polymerfilm und dem diesem benachbarten gummielastischen Stopfenteil im Durchstichbereich für einen Infusionsdorn od. dgl. unterbrochen ist.

Dabei hat sich die Erfindung die Erkenntnis zunutze gemacht, daß bei den bekannten Stopfen der eingangs erwähnten Art der Infusionsdorn den Polymerfilm od. dgl. Inertfilm an der Durchstichstelle unter Bildung eines Durchstichschlitzes in Richtung des Behälterinneren verbiegt und der fluorierte Polymerfilm od. dgl. Inertfilm nach Entfernen des Infusionsdornes in etwa die nach dem Durchstechen des Infusionsdornes eingenommene Form beibehält. Der entstandene Schlitz wird somit wegen mangelnder Elastizität des Polymerfilmes durch diesen offengehalten.

Der erfindungsgemäße Stopfen hat den Vorteil, daß sich der Stopfenhals in der üblichen Weise nach dem Entfernen des Infusionsdornes wieder etwa so schließen kann, wie er es auch ohne Vorhandensein des Polymerfilmes od. dgl. Inertfilm tun würde. Aufgrund der erfindungsgemäßen Lösung kann man z.B. den Flascheninhalt auch nach dem Durchstechen schütteln, und selbst wenn sich im Flascheninnern bei Zugabe einer Flüssigkeit oder eines Medikamentenanteiles und/oder beim Schütteln ein Überdruck ergibt, erhält man noch einen ausreichend sicheren und lagerfähigen Verschluß der Medikamentenflasche.

Die Erfindung betrifft auch ein Verfahren zur Herstellung des erfindungsgemäßen Stopfens. Die Erfindung eignet sich insbesondere für eine Weiterentwicklung eines Verfahrens zur Herstellung von Stopfen od. dgl. Gummiteil zum Verschließen einer Flasche, eines Spritzenzylinders od. dgl. Behälter, wobei der Stopfen od. dgl. in einem dem Behälterinnenraum zugewandten, mindestens teilweise an der Behälterinnenwand anliegenden Bereich einen fluorierten Polymer-od. dgl. Inertfilm aufweist und der Stopfenhals mit einem weiteren Bereich unmittelbar an der Wand der Behältermündung anliegt, und wobei Kautschukmaterial im Formwerkzeug eingebracht sowie dort vulkanisiert wird (vgl. EP-Anmeldung Nr. 84 114 893.5). Die Erfindung besteht darin, daß die Haftung zwischen Polymerfilm od. dgl. Inertfilm und dem gummielastischen Teil des Stopfeninnenteiles im Bereich der Durchstichstelle eines Infusions dornes unterbrochen wird.

Dies kann in Ausgestaltung der Erfindung z. B. dadurch geschehen, daß die Haftfähigkeit des Polymerfilmes im Bereich der Durchstichstelle eines Infusionsdornes beseitigt wird. Dadurch bleibt an dieser Stelle die Verbindung zwischen dem einvulkanisierten Gummiteil, also dem Stopfeninnenteil, und der schützenden Außenhaut des Polymerfilmes unterbrochen. Dieses Verfahren ist unkompliziert und erfordert keine grundsätzliche Änderung der Art und Weise der Herstellung dieser Stopfen (vgl. EP-Anmeldung Nr. 84 114 893.5). Um den angestrebten Effekt zu erreichen, kann man z. B. die Polymerfilmfolie od. dgl., Inertfilm entsprechend vorbehandeln.

Diese Vorbehandlung kann in einer Ausgestaltung der Erfindung auf einfache Weise realisiert werden, indem eine die Oberflächenstruktur des fluorierten Polymerfilmes od. dgl. Inertfilmes haftfähig machende Ätzung oder Beschichtung mit entsprechendem Kleber vor dem Verbinden des Polymerfilmes mit dem Kautschukmaterial od. dgl. im Bereich der späteren Durchstichstelle für ein Infusionsdorn od. dgl. entfernt wird. Dieses Vorgehen ist u. a. deshalb vorteilhaft, weil der übliche Herstellungsprozeß nur leicht modifiziert zu werden braucht. Denn der vorzugsweise aus PTFE bestehende Inertfilm wird normalerweise durch Ätzen seiner späteren Verbindungsfläche zunächst im ganzen haftfähig gemacht, da die Ätzung eine dafür geeignete Oberflächenstruktur ermöglicht. Ohne diese Ätzung haftet der Film nicht. In der hier beschriebenen Ausgestaltung des erfindungsgemäßen Verfahrens kann man sich also darauf beschränken, durch einen einzigen zusätzlichen Verfahrensschritt die Ätzung punktuell wieder rückgängig zu machen.

Dies geschieht vorzugsweise mittels Erhitzen der Durchstichstelle des Polymerfilmes.

In einer anderen Ausgestaltung der Erfindung werden im Bereich der Durchstichstelle räumlich genau bemessene Portionen einer Chemikalie aufgebracht, welche bei den folgenden Verfahrensschritten eine Haftfähigkeit des Inertfilmes an den vorgesehenen Stellen beseitigt.

Nachstehend wird die Erfindung mit ihren wesentlichen Einzelheiten an Hand von Ausführungsbeispielen in Verbindung mit der Zeichnung näher beschrieben. Es zeigt:

Fig. 1 Einen Längsschnitt durch einen Stopfen einer Medikamentenflasche, durch den ein Infusionsdorn gesteckt ist,

Fig. 2 einen Längsschnitt durch einen Stopfen entsprechend Fig. 1, aus dem der Infusionsdorn herausgezogen ist,

Fig.3a Stopfenunterteile, bei denen die Flächenverbindung und 3b zwischen dem fluorierten Polymerfilm und dem gummielastischen Stopfenteil im Durchstichbereich für einen Infusionsdorn unterbrochen ist,

Fig. 4 einen Teil-Längsschnitt einer offenen Herstellungsform, jedoch für eine etwas abgewandelte Herstellungsweise und

Fig. 5 die Herstellungsform nach Figur 5 in Schließposition.

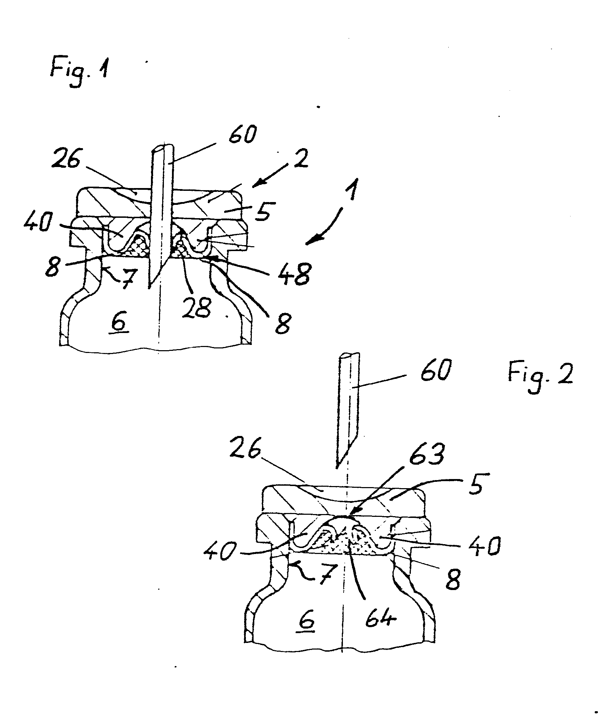

Fig. 1 zeigt einen Stopfen 2 auf einer Medikamentenflasche 1, die von einem Infusionsdorn 60 durchstochen ist. Der Stopfen 2 ist in seinem Innern mit der dem Behälterinnen raum 6 zugewandten Seite mit einem Polymerfilm 8 ausgekleidet. Der Stopfen 2 besitzt auf seiner äußeren und seiner inneren Stirnseite Aussparungen 26 bzw. 28, damit die Durchstichlänge beim Einstechen einer Injektionsnadel, eines Infusionsdornes 60 od. dgl. verkürzt wird. Die Aussparung 28 auf der inneren Stirnseite des Stopfens 2 ist hierbei vollständig mit einem Polymerfilm 8 ausgekleidet.

Deutlich erkennbar ist in Fig. 1, daß der Polymerfilm 8 an der Durchstichstelle durch den Infusionsdorn 60 verformt ist. Unter Bildung eines Durchstich-Schlitzes verbiegt sich der Polymerfilm 8 od. dgl. in Richtung des Behälterinneren 6. Entfernt man den Infusionsdorn 60, wie in Fig. 2 erkennbar, behält der fluorierte Polymerfilm 8 od. dgl. in etwa die nach dem Durchstich des Infusionsdornes 60 eingenommene Form bei. In Fig. 2 ist dabei auch der im Polymerfilm 8 entstandene Schlitz 64 bei der früheren Durchstichstelle des Infusionsdornes 60 gut erkennbar. Die Polymerschicht 8 kann, wenn sie im Bereich der Durchstichstelle fest mit dem gummielastischen Werkstoff des Stopfenteiles 40 in Verbindung steht, diese gummielastische Duchstichstelle 63 zumindest bereichsweise, im wesentlichen im Nachbarbereich des Polymerfilmes 8, etwas offenhalten. Neben dem bereits erwähnten Mangel an Elastizität des Polymerfilmes dürfte dieses Verhalten vor allem durch eine starke Verdehnung des fluorierten Polymerfilm 8 od. dgl. bedingt sein durch den nachstehend beschriebenen Herstellungsprozeß. Das durch den Infusionsdorn 60 verursachte Aufplatzen des Polymerfilmes 8 führt deshalb dazu, daß er, unter Spannung stehend, sich ein Stück weit in Richtung des Stopfenrandes 68 zusammenzieht und damit das mit ihm verbundene gummielastische Material des Stopfenteiles 40 daran hindert, in seine ursprüngliche Lage zurückzugehen und den entstandenen Schlitz zu - schließen.

Der hier beschriebene Effekt wird vermieden, wenn im Bereich der Durchstichstelle 63 die Verbindung zwischen Polymerfilm und Stopfenteil unterbrochen ist. Verformungen des Polymerfilms 8 wirken sich nicht mehr auf den benachbarten gummielastischen Bereich des Stopfens aus, der einerseits das durch den Infusionsdorn 60 entstandene Loch wieder dicht verschließen kann.

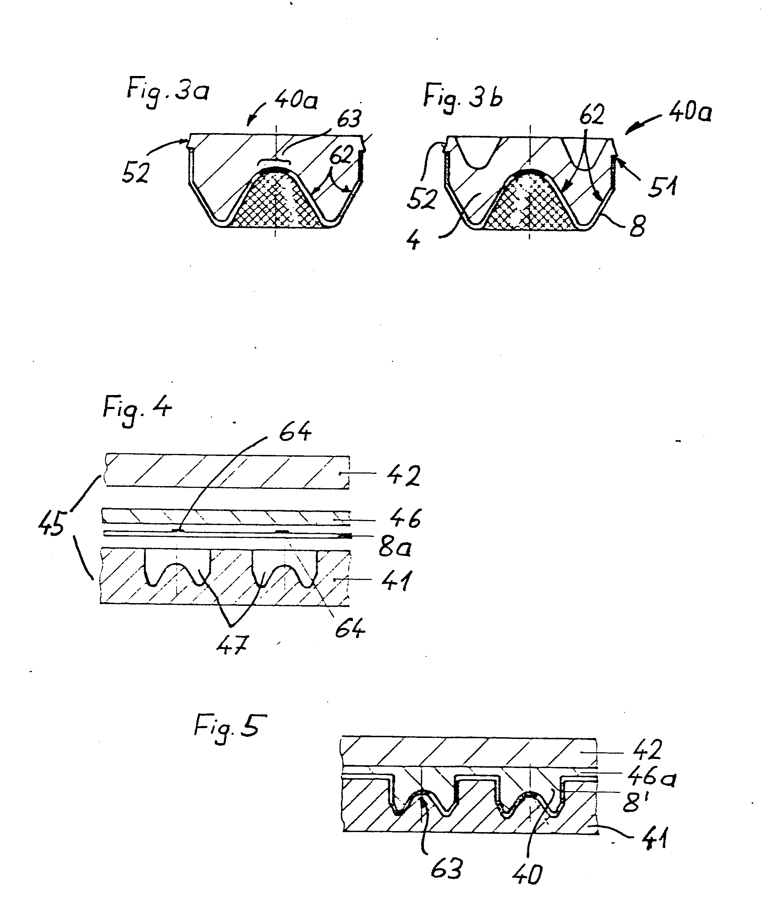

Das im folgenden beispielhaft beschriebene Verfahren zur Herstellung des erfindungsgemäßen Stopfens lehnt sich an das in der EP-Anmeldung Nr. 84 114 893.5 beschriebene Verfahren an. Für die Herstellung der Verbindung des Polymerfilms 8 mit dem gummielastischen Stopfenteil 40 ist eine erste Kaliberplatte 41 (Fig. 4) vorgesehen, die mit einer ersten Gegenplatte 42 das erste Formwerkzeug 45 für den ersten Arbeitsgang bildet. Zwischen die geöffneten Teile 41, 42 des ersten Formwerkzeuges 45 sind, in Fig. 5 stark schematisiert dargestellt, ein unvulkanisiertes Gummifell 46" und ein unverformter, flacher Polymerfilm 8a angeordnet. Unter "Gummifell" versteht man in der einschlägigen Fachwelt eine Gummischicht von einer gewissen Längen-und Breitenausdehnung, mit dem gewöhnlich eine Form an den Rändern so weit überdeckt werden kann, daß ein Arbeitsgang möglich ist. Der zunächst noch ebene, folienähnliche fluorierte Polymerfilm 8a (Fig. 4) kann sich bei diesem Herstellungsschritt bereits in fester Verbindung mit dem Gummifell 46 befinden oder er kann, wie Fig. 4 schematisch zeigt, unabhängig und mit einem Abstand vom Gummifell 46 zwischen die Formteile 41 und 42 eingebracht werden. Das getrennte Einbringen von Gummifell 46 und ebenem fluorierten Polymerfilm 8a hat unter anderem den Vorteil, daß ein besonderer Arbeitsgang zum Verbinden von Gummifell 46 und fluorisiertem Polymerfilm 8a eingespart werden kann.

Fig. 5 zeigt das in Fig. 4 dargestellte erste Formwerkzeug in der Schließposition. Hier ist das zunächst noch unvulkanisierte, jetzt mit 46a bezeichnete Gummifell in die dem herzustellenden Stopfenteil 40 entsprechenden Kaliberformen 47 eingedrückt worden. Dabei ist der fluorierte, zunächst ebene Polymerfilm 8a in die gewünschte napfartige Form gebracht worden, wobei er im Unterschied zu Fig. 5 mit 8' bezeichnet ist. Wird das Formwerkzeug 45 unter Druck geschlossen, wird der unvulkanisierte Gummifell-Werkstoff zusammen mit dem fluorisierten Polymerfilm 8a in die Kaliberform 47 eingepreßt. Dadurch wird der fluorisierte Polymerfilm 8a od. dgl. tiefgezogen, wie gut aus Fig. 5 erkennbar.

Das Vorbereiten einer Polymerfolie 8 od. dgl. zum Erreichen einer mechanischen Verbindung gegenüber dem gummielastischen Teil des Stopfens 2 od. dgl. erfolgt gewöhnlich auf chemische Weise, wobei die entsprechende Oberfläche der Polymerfolie 8 genügend "griffig" gemacht wird, so daß sich der gummielastische Werkstoff -im Mikrobereich gesehen-auf mechanische Weise durch Verhaken, Hintergreifen usw. fest mit dem Polymerfilm 8 verbinden kann. Zu diesem Zweck wird der vorzugsweise aus PTFE bestehende Inertfilm 8 durch Ätzen seiner späteren Verbindungsfläche 62 haftfähig gemacht.

Um den erfindungsgemäßen Stopfen herzustellen, wird im Ausführungsbeispiel gemäß Fig. 3a und 3b die durch Ätzen hervorgerufene Flächenstruktur, im Bereich der Durchstichstelle 63 des Infusionsdornes 60 wieder aufgehoben. Dann verbindet sich die PTFE-Folie 8 in diesem Bereich nicht mit dem gummielastischen Werkstoff des Stopfenteiles 40. Die besondere Ausbildung des Inertfilmes 8 an der Durchstichstelle 63 ist in den Fig. 3, 3a, 4, 5 durch die ausgezogene schwarze Linie im Bereich 63 angedeutet. Für das Verfahren zur Herstellung der Haftunfähigkeit des Polymerfilms an der geforderten Durchstichstelle 63 bieten sich vorzugsweise zwei Möglichkeiten an. Der bandförmig oberhalb der Kaliberplatte 41 befindliche fluorierte Polymerfilm 8a kann beispielsweise durch chemische Zugaben an der späteren Durchstichstelle 63 so behandelt werden, daß dort bei der Herstellung des Stopfenteiles 40 keine Verbindung zwischen dem gummielastischem Werkstoff des Stopfenteiles 40 einerseits und der Verbindungsfläche 62 des Polymerfilmes 8a auftritt. In Fig. 4 sind dazu z.B. räumlich genau bemessene Portionen 64 einer Chemikalie aufgebracht, welche beim Herstellungsvorgang eine dort unerwünschte Haftfähigkeit des Inertfilmes 8 bzw. 8a verhindert.

Eine andere Variante besteht darin, den Polymerfilm 8 vor dem Einpressen der Polymerfolie in den Bereichen der Durchstichstellen 63 zu erhitzen. Dadurch wird die durch Ätzen vorher hervorgerufene Flächenstruktur wieder aufgehoben, so daß sich auch nach diesem Verfahren der Polymerfilm 8 nicht mehr mit dem gummielastischen Werkstoff des Stopfenteiles 40 verbinden kann.

Nach Beendigung des Preßvorganges wird das zumindest anvulkanisierte, gegebenenfalls ausvulkanisierte Gummifell 46a, auf dessen einer Seite sich der fluorierte Polymerfilm 8 befindet, der nun in den Bereichen der späteren Durchstichsstellen eines Infusionsdornes 60 keine Verbindung mit dem gummielastischen Teil des Stopfens 2 eingegangen ist, aus dem Formwerkzeug 45 entfernt, und die auf diese Weise hergestellten Stopfenteile werden sodann ausgestanzt. Das weitere Verfahren zur Herstellung des Stopfens betrifft nicht mehr den Gegenstand der vorstehenden Erfindung. Hierzu wird auf die Beschreibung in der EP-Anmeldung Nr. 84 114 893.5 verwiesen.

## Ansprüche

1. Pharmazeutischer Stopfen (2) zum Verschließen einer Flasche, eines medizinischen, pharmazeutischen od. dgl. Behälters, insbesondere einer Medikamentenflasche (1), wobei der Stopfen (2) in einem dem Behälterinnenraum (6) zugewandten, mindestens teilweise an der Behälterinnenwand (7) anliegenden Bereich einen fluorierten Polymer-oder dgl. Inertfilm (8) aufweist und der Stopfenhals mit einem weiteren Bereich unmittelbar an der Wand der Behältermündung anliegt, **dadurch gekennzeichnet,** daß die Flächenverbindung zwischen dem fluorierten Polymerfilm (8) und dem diesem benachbarten gummielastischen Stopfenteil (40) im Durchstichbereich für einen Infusionsdorn (60) od. dgl. unterbrochen ist.

2. Verfahren zur Herstellung von Stopfen nach Anspruch 1, dadurch gekennzeichnet, daß Haftung zwischen Polymerfilm (8) od. dgl. Inertfilm und dem gummielastischen Stopfeninnenteil (40) im Bereich der Durchstichstelle (63) eines Infusionsdornes (60) unterbrochen wird.

3. Verfahren nach Anspruch 2, dadurch gekennzeichnet, daß eine die Oberflächenstruktur des fluorierten Polymerfilmes (8) haftfähig machende Ätzung oder Beschichtung mit entsprechendem Kleber vor dem Verbinden des Polymerfilmes (8) mit dem Kautschukmaterial od. dgl. im Bereich der späteren Durchstichstelle (63) für einen Infusionsdorn (60) od. dgl. entfernt wird.

4. Verfahren nach Anspruch 2 oder 3, dadurch gekennzeichnet, daß im Durchstichbereich eines Infusionsdornes (60) auf den Polymerfilm (8) od. dgl. Inertfilm räumlich genau bemessene Portionen einer Chemikalie aufgebracht werden, welche beim Herstellungsvorgang eine Haftfähigkeit des Inertfilmes (8) verhindert.

5. Verfahren nach Anspruch 2 oder 3, dadurch gekennzeichnet, daß der Polymer-oder dgl. Inertfilm (8) im Durchstichbereich eines Infusionsdornes (60) erhitzt wird.

Fig. 1

Fig. 2

Fig. 3a

40a

63

62

52

Fig. 3b

62

40a

52

51

4

8

Fig. 4

64

42

46

8a

41

45

47

64

Fig. 5

42

46a

8'

41

63

40

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | FR-A-2 074 704 (RUMPLER) * Seite 2, Zeilen 31-39; Seite 3, Zeile 21 - Seite 4, Zeile 19; Anspruch 6; Abbildungen 1-4 * | 1,2 | B 65 D 51/00 |
| A | | 3 | |
| | --- | | |
| Y | NL-A-6 817 622 (SCHERICO LTD.) * Seite 4, Zeile 3 - Seite 5, Zeile 3; Abbildungen 1,3 * | 1,2 | |
| | --- | | |
| A | FR-A-1 136 064 (S.E.S.I.) * Seite 1, rechte Spalte, Zeilen 19-32; Abbildungen 1,2 * | 3 | |
| | --- | | |
| A,D | DE-A-2 146 421 (TAKEDA CHEMICAL IND.) * Seite 7, Zeile 14 - Seite 9, Zeile 11; Abbildungen 1-4 * | 1 | |
| | ----- | | |

RECHERCHIERTE SACHGEBIETE (Int. Cl.4)

A 61 J
B 29 C
B 65 D

Der vorliegende Recherchenbericht wurde für alle Patentansprüche erstellt.

| Recherchenort | Abschlußdatum der Recherche | Prüfer |
|---|---|---|
| DEN HAAG | 24-11-1986 | BERRINGTON N.M. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82